Europäisches Patentamt

European Patent Office

Office européen des brevets

(11) Publication number : **0 302 836 B1**

# EUROPEAN PATENT SPECIFICATION

(45) Date of publication of patent specification :
**11.03.92 Bulletin 92/11**

(51) Int. Cl.⁵ : **A61K 31/71, A61K 47/08**

(21) Application number : **88830287.4**

(22) Date of filing : **07.07.88**

(54) **Pharmaceutical compositions for topical use containing miocamycin.**

The file contains technical information
submitted after the application was filed and
not included in this specification

(73) Proprietor : **A. MENARINI INDUSTRIE
FARMACEUTICHE RIUNITE S.R.L.
Via Sette Santi 3
I-50131 Firenze (IT)**

(30) Priority : **08.07.87 IT 943087**

(72) Inventor : **Ghelardoni, Mario
Via R. Lambruschini No. 5
Firenze (IT)**
Inventor : **Bani, Raffaello
Via Val di Chiana No. 56
Firenze (IT)**
Inventor : **Carlesi, Roberto Massimo
Via Caravina No. 7
Civenna Como (IT)**
Inventor : **Melani, Francesco
Via Poggio Magherini No. 5
Fiesole Firenze (IT)**

(43) Date of publication of application :
**08.02.89 Bulletin 89/06**

(45) Publication of the grant of the patent :
**11.03.92 Bulletin 92/11**

(84) Designated Contracting States :
**AT BE CH DE ES FR GB GR LI LU NL SE**

(56) References cited :
**EP-A- 0 135 617
GB-A- 2 132 086
US-A- 4 335 115
Martindale, The Extra Pharmacopoeia, 1982.**

(74) Representative : **Mannucci, Gianfranco,
Dott.-Ing.
Ufficio Tecnico Ing. A. Mannucci Via della
Scala 4
I-50123 Firenze (IT)**

EP 0 302 836 B1

Note : Within nine months from the publication of the mention of the grant of the European patent, any
person may give notice to the European Patent Office of opposition to the European patent granted.
Notice of opposition shall be filed in a written reasoned statement. It shall not be deemed to have been
filed until the opposition fee has been paid (Art. 99(1) European patent convention).

## Description

The invention relates to the preparation of pharmaceutical forms containing miocamycin for topical administration (ointment, gel, globuli, ophthalmic ointment, and mouthwash), for the therapy of bacterial infections of the skin and of the mucous membranes (conjunctival, oropharyngeal, and vulvo-vaginal-cervical).

The antibiotic therapy of bacterial infections of the skin and of the mucous membranes, as well as those dermatological disorders in which a microbial etiological aspect is involved (for example, acne), makes use, depending upon the particular cases involved, not only of the systemic administration route but also - and on occasions exclusively - of the topical administration route. Various chemo-antibiotics such as tetracyclines, aminoglycosides, chloramphenicol, are, in fact, widely used via the topical route.

Miocamycin, a macrolide antibiotic with a spectrum of action such as to justify the use thereof in the infections set forth above (Yoshida et al., Jap. J. Antibiotics 135, 1462, 1982), although representing, as compared with other pharmaceutical products, an advantageous alternative both on account of the high degree of efficacy and on account of the excellent tolerability, has, to date, never been used for topical administration in the case of the abovementioned disorders.

GB-A-2132086 discloses oral preparations of macrolide antibiotics, and in particular also of miocamycin. These oral preparations, however, are not suitable for administration through the topical route. In particular, no pharmaceutical forms containing excipients suitable for absorption through the skin are described. Rather, solid (granular) pharmaceutical forms suitably stabilized for enhancing bioavailability when administered through the oral route are disclosed.

According to Martindale (Extra Pharmacopoeia 1982, page 1185 and page 1158), miocamycin is closely related to midecamycin, midecamycin has actions similar to those of erythromycin, and topical pharmaceutical forms like ointments and ophtalmic ointments having erythromycin as an active principle are known.

The invention refers to a stable pharmaceutical composition containing miocamycin as active principle, and a carrier suitable for topical administration, for the therapy of bacterial infections of the skin and of the mucous membranes. In order to have a composition stable for a long period of time, the composition does not contain water. In those cases, such as mouthwash or gel forms, in which water is added to the composition, this is done only at the time of use.

Examples of the formulation of various pharmaceutical forms according to the invention for topical administration, are given herein below:

Example 1

An oinment having the following composition, related to 100 g:

| Miocamycin | from | 1 | to | 5 | g |
|---|---|---|---|---|---|
| Ethylcellulose | from | 0.08 | to | 0.4 | g |
| Non-ionic surfactants | from | 3 | to | 7 | g |
| Aliphatic mixed esters | from | 50 | to | 80 | g |
| Glycerides of saturated fatty acids | from | 5 | to | 15 | g |
| Preservatives | from | 0.05 | to | 0.2 | g |

Example 2

The pharmaceutical form is a gel and consists in a suitable bottle containing the excipient and provided with a reservoir cap containing the active principle to be combined with the excipient at the time of use. This is due to the poor stability of the active principle over a long period of time in the presence of water; the gel thickens on being administered to the tissues at body temperature.

The composition is the following, related to 100 g:

Content of the bottle:

EP 0 302 836 B1

| | |
|---|---|
| Hydroxypropylmethylcellulose | from 0.5 to 3 g |
| Poloxamers and/or poloxamines | from 5 to 50 g |
| Preservatives | from 0.05 to 0.2 g |
| Buffering substances q.s. at pH 7 - 7.5 | |
| Water q.s. | to 100 g |
| Content of the reservoir cap: | |
| Miocamycin | from 1 to 5 g |
| Ethylcellulose | from 0.08 to 0.4 g |

Example 3

Globuli having the following unit composition:

| | |
|---|---|
| Miocamycin | from 0.25 to 0.8 g |
| Ethylcellulose | from 0.02 to 0.07 g |
| Non-ionic surfactants | from 0.05 to 0.15 g |
| Polyethylene glycols | from 3 to 6 g |
| Propylene glycol | from 0 to 2 g |

Example 4

Globuli having the following unit composition:

| | |
|---|---|
| Miocamycin | from 0.25 to 0.8 g |
| Ethylcellulose | from 0.02 to 0.07 g |
| Semisynthetic glycerides of saturated fatty acids | from 3 to 6 g |

Example 5

Mouthwash consisting of granules in a single-dose package, to be suspended in water at the time of use, and having the following unit composition:

| | |
|---|---|
| Miocamycin | from 0.3 to 0.9 g |
| Ethylcellulose | from 0.024 to 0.08 g |
| Hydroxypropylmethylcellulose | from 0.15 to 0.3 g |
| Mannitol | from 1.5 to 4 g |
| Flavorings | from 0.05 to 0.15 g |
| Dimethicone | from 0.0005 to 0.001 g |
| Non-ionic surfactants | from 0.0005 to 0.001 g |
| Synthetic sweetener | from 0.005 to 0.05 g |

3

Example 6

Ophthalmic ointment having the following composition per 100 g:

| Miocamycin | from 1 | to 10 | g |
|---|---|---|---|
| Ethylcellulose | from 0.08 | to 0.8 | g |
| Vegetable oils | from 20 | to 30 | g |
| Solid paraffin | from 40 | to 60 | g |
| Non-ionic surfactants | from 0.5 | to 5 | g |
| Polyoxyethylenated oleic glycerides | from 5 | to 10 | g |
| Preservatives | from 0.05 | to 0.2 | g |

## Claims

### Claims for the following Contracting States : AT, BE, CH, DE, FR, GB, LI, LU, NL, SE

1. A stable pharmaceutical composition containing miocamycin as active principle and a carrier suitable for topical administration, in the absence of water, for the therapy of bacterial infections of the skin and of the mucous membranes (conjunctival, oropharyngeal, and vulvo-vaginal-cervical).

2. The composition as claimed in claim 1 in ointment form, having a formulation as follows, related to 100 g:

| Miocamycin | from 1 | to 5 | g |
|---|---|---|---|
| Ethylcellulose | from 0.08 | to 0.4 | g |
| Non-ionic surfactants | from 3 | to 7 | g |
| Aliphatic mixed esters | from 50 | to 80 | g |
| Glycerides of saturated fatty acids | from 5 | to 15 | g |
| Preservatives | from 0.05 | to 0.2 | g |

3. The composition as claimed in claim 1, in the form of globuli having the following unit formulation:

| Miocamycin | from 0.25 | to 0.8 | g |
|---|---|---|---|
| Ethylcellulose | from 0.02 | to 0.07 | g |
| Non-ionic surfactants | from 0.05 | to 0.15 | g |
| Polyethylene glycols | from 3 | to 6 | g |
| Propylene glycol | from 0 | to 2 | g |

4. The composition as claimed in claim 1, in the form of globuli having the following unit formulation:

| Miocamycin | from 0.25 to 0.8 g |
| Ethylcellulose | from 0.02 to 0.07 g |
| Semisynthetic glycerides of saturated fatty acids | from 3 to 6 g |

5. The composition as claimed in claim 1, in the form of an ophthalmic ointment having the following formulation per 100 g:

| Miocamycin | from 1 to 10 g |
| Ethylcellulose | from 0.08 to 0.8 g |
| Vegetable oils | from 20 to 30 g |
| Solid paraffin | from 40 to 60 g |
| Non-ionic surfactants | from 0.5 to 5 g |
| Polyoxyethylenated oleic glycerides | from 5 to 10 g |
| Preservatives | from 0.05 to 0.2 g |

6. A pharmaceutical composition, containing miocamycin as an active principle for topical administration in the form of mouthwash, consisting of granules in a single-dose packet, to be suspended in water at the time of use, having the following unit formulation:

| Miocamycin | from 0.3 to 0.9 g |
| Ethylcellulose | from 0.024 to 0.08 g |
| Hydroxypropylmethylcellulose | from 0.15 to 0.3 g |
| Mannitol | from 1.5 to 4 g |
| Flavorings | from 0.05 to 0.15 g |
| Dimethicone | from 0.0005 to 0.001 g |
| Non-ionic surfactants | from 0.0005 to 0.001 g |
| Synthetic sweetener | from 0.005 to 0.05 g |

7. A pharmaceutical composition containing miocamycin for topical administration for the therapy of bacterial infections of the skin and of the mucous membranes consisting in a bottle containing water and a gelling agent, and a cap containing the miocamycin as active principle, the active principle being mixed with the content of the bottle at the time of use.

8. The composition as claimed in claim 7, having a formulation as follows, related to 100 g:
Content of the bottle:

| Hydroxypropylmethylcellulose | from 0.5 to 3 g |
| Poloxamers and/or poloxamines | from 5 to 50 g |
| Preservatives | from 0.05 to 0.2 g |
| Buffering substances q.s. at pH 7 - 7.5 | |

5

| | | |
|---|---|---|
| Water q.s. | to 100 | g |
| Content of the reservoir cap: | | |
| Miocamycin | from 1 to 5 | g |
| Ethylcellulose | from 0.08 to 0.4 | g |

**Claims for the following Contracting States : GR, ES**

1. Method for the preparation of a stable pharmaceutical composition containing miocamycin as active principle, wherein the active principle is added to a carrier suitable for topical administration, in the absence of water, for the therapy of bacterial infections of the skin and of the mucous membranes (conjunctival, oropharyngeal, and vulvo-vaginal-cervical).

2. The method as claimed in claim 1, for the preparation of an ointment having a formulation as follows, related to 100 g:

| | | |
|---|---|---|
| Miocamycin | from 1 to 5 | g |
| Ethylcellulose | from 0.08 to 0.4 | g |
| Non-ionic surfactants | from 3 to 7 | g |
| Aliphatic mixed esters | from 50 to 80 | g |
| Glycerides of saturated fatty acids | from 5 to 15 | g |
| Preservatives | from 0.05 to 0.2 | g |

3. The method as claimed in claim 1, for the preparation of a pharmaceutical composition in form of globuli having the following unit formulation:

| | | |
|---|---|---|
| Miocamycin | from 0.25 to 0.8 | g |
| Ethylcellulose | from 0.02 to 0.07 | g |
| Non-ionic surfactants | from 0.05 to 0.15 | g |
| Polyethylene glycols | from 3 to 6 | g |
| Propylene glycol | from 0 to 2 | g |

4. The method as claimed in claim 1, for the preparation of a pharmaceutical composition in the form of globuli having the following unit formulation:

| | | |
|---|---|---|
| Miocamycin | from 0.25 to 0.8 | g |
| Ethylcellulose | from 0.02 to 0.07 | g |
| Semisynthetic glycerides of saturated fatty acids | from 3 to 6 | g |

5. The method as claimed in claim 1, for the preparation of a pharmaceutical composition in the form of an ophthalmic ointment having the following formulation per 100 g:

| Miocamycin | from | 1 | to 10 | g |
| Ethylcellulose | from | 0.08 | to 0.8 | g |
| Vegetable oils | from | 20 | to 30 | g |
| Solid paraffin | from | 40 | to 60 | g |
| Non-ionic surfactants | from | 0.5 | to 5 | g |
| Polyoxyethylenated oleic glycerides | from | 5 | to 10 | g |
| Preservatives | from | 0.05 | to 0.2 | g |

6. A method for the preparation of a pharmaceutical composition, containing miocamycin as an active principle, wherein the active principle is added to a carrier suitable for topical administration, in the form of a mouthwash, consisting of granules in a single-dose packet, to be suspended in water at the time of use, having the following unit formulation:

| Miocamycin | from 0.3 | to 0.9 | g |
| Ethylcellulose | from 0.024 | to 0.08 | g |
| Hydroxypropylmethylcellulose | from 0.15 | to 0.3 | g |
| Mannitol | from 1.5 | to 4 | g |
| Flavorings | from 0.05 | to 0.15 | g |
| Dimethicone | from 0.0005 | to 0.001 | g |
| Non-ionic surfactants | from 0.0005 | to 0.001 | g |
| Synthetic sweetener | from 0.005 | to 0.05 | g |

7. A method for the preparation of a pharmaceutical composition containing miocamycin as the active principle, suitable for topical administration for the therapy of bacterial infections of the skin and of the mucous membranes, wherein the active principle is inserted in a cap of a bottle, the bottle containing water and a gelling agent, the active principle being mixed with the content of the bottle at the time of use.

8. The method as claimed in claim 7, for the preparation of a composition having a formulation as follows, related to 100 g:

Content of the bottle:

| Hydroxypropylmethylcellulose | from 0.5 | to 3 | g |
| Poloxamers and/or poloxamines | from 5 | to 50 | g |
| Preservatives | from 0.05 | to 0.2 | g |
| Buffering substances q.s. at pH 7 - 7.5 | | | |
| Water q.s. | | to 100 | g |

Content of the reservoir cap:

| Miocamycin | from 1 | to 5 | g |
| Ethylcellulose | from 0.08 | to 0.4 | g |

**Patentansprüche**

**Patentansprüche für folgenden Vertragsstaate: AT, BE, CH, DE, FR, GB, LI, LU, NL, SE**

1. Stabile pharmazeutische Zusammensetzung, die Miocamycin als Wirkstoff und einen Träger enthält, der, in Abwesenheit von Wasser für eine äußerliche Anwendung geeignet ist, für die Behandlung bakterieller Infektionen der Haut und der Schleimhäute (Bindehaut, Mund- und Rachenschleimhaut, Vulvo-Vaginal-Zervical-Schleimhaut),

2. Zusammensetzung nach Anspruch 1 in Salbenform, die, bezogen auf 100 g, folgende Rezeptur aufweist:

| | | | | | |
|---|---|---|---|---|---|
| Miocamycin | von | 1 | bis | 5 | g |
| Äthylzellulose | von | 0,08 | bis | 0,4 | g |
| Nicht-ionische Tenside | von | 3 | bis | 7 | g |
| Aliphatische Mischester | von | 50 | bis | 80 | g |
| Glyzeride gesättigter Fettsäuren | von | 5 | bis | 15 | g |
| Konservierungsmittel | von | 0,05 | bis | 0,2 | g |

3. Zusammensetzung nach Anspruch 1, in Form von Kügelchen (Globuli), welche die folgende Einheitenrezeptur aufweist:

| | | | | | |
|---|---|---|---|---|---|
| Miocamycin | von | 0,25 | bis | 0,8 | g |
| Ähtylzellulose | von | 0,02 | bis | 0,07 | g |
| Nicht-ionische Tenside | von | 0,05 | bis | 0,15 | g |
| Polyäthylenglycole | von | 3 | bis | 6 | g |
| Propylenglycol | von | 0 | bis | 2 | g |

4. Zusammensetzung nach Anspruch 1, in Form von Kügelchen (Globuli), welche die folgende Einheitenrezeptur aufweist:

| | | | | | |
|---|---|---|---|---|---|
| Miocamycin | von | 0,25 | bis | 0,8 | g |
| Ähtylzellulose | von | 0,02 | bis | 0,07 | g |
| Halbsynthetische Glyzeride von gesättigten Fettsäuren | von | 3 | bis | 6 | g |

5. Zusammensetzung nach Anspruch 1 in Form einer Augensalbe, die, bezogen auf 100 g, folgende Rezeptur aufweist:

| | | | | | |
|---|---|---|---|---|---|
| Miocamycin | von | 1 | bis | 10 | g |
| Äthylzellulose | von | 0,08 | bis | 0,8 | g |
| Pflanzliche Öle | von | 20 | bis | 30 | g |
| Festes Parafin | von | 40 | bis | 60 | g |
| Nicht-ionische Tenside | von | 0,5 | bis | 5 | g |
| Polyoxyäthylenierte Ölsäureglyzeride | von | 5 | bis | 10 | g |
| Konservierungsmittel | von | 0,05 | bis | 0,2 | g |

6. Pharmazeutische Zusammensetzung, die Miocamycin als Wirkstoff für eine äußerliche Anwendung in Form einer Mundspülung enthält und die aus einem Granulat in einem Einzeldosispaket besteht, das zum Zeitpunkt des Gebrauches in Wasser zu suspendieren ist und die die folgende Einheitsrezeptur aufweist:

| | | | | | |
|---|---|---|---|---|---|
| Miocamycin | von | 0,3 | bis | 0,9 | g |
| Äthylzellulose | von | 0,024 | bis | 0,08 | g |
| Hydroxypropylmethyl-zellulose | von | 0,15 | bis | 0,3 | g |
| Mannit | von | 1,5 | bis | 4 | g |
| Geschmacksstoffe | von | 0,05 | bis | 0,15 | g |
| Dimethicone | von | 0,0005 | bis | 0,001 | g |
| Nicht-ionische Tenside | von | 0,0005 | bis | 0,001 | g |
| Synthetischer Süßstoff | von | 0,005 | bis | 0,05 | g |

7. Pharmazeutische Zusammensetzung, die Miocamycin als Wirkstoff für äußerliche Anwendung für die Behandlung bakterieller Infektionen der Haut und der Schleimhäute enthält und die aus einer Wasser und ein Geliermittel enthaltenden Flasche und einer Kappe besteht, welche das Miocamycin als Wirkstoff enthält, wobei der Wirkstoff mit dem Inhalt der Flasche zur Gebrauchszeit gemischt wird.

8. Zusammensetzung nach Anspruch 7, welche folgende, auf 100 g bezogene Rezeptur hat:

Inhalt der Flasche:

| | | | | | |
|---|---|---|---|---|---|
| Hydroxypropylmethylzellulose | von | 0,5 | bis | 3 | g |
| Poloxamere und/oder Poloxamine | von | 5 | bis | 50 | g |
| Konservierungsmittel | von | 0,05 | bis | 0,2 | g |
| Puffersubstanzen ad pH 7 - 7,5 | | | | | |
| Wasser | | | ad | 100 | g |

Inhalt der Behälterkappe:

| | | | | | |
|---|---|---|---|---|---|
| Miocamycin | von | 1 | bis | 5 | g |
| Äthylzellulose | von | 0,08 | bis | 0,4 | g |

**Patentansprüche für folgenden Vertragsstaate: GR, ES**

1. Verfahren zur Herstellung einer stabilen pharmazeutischen Zusammensetzung, die Miocamycin als Wirkstoff enthält, wobei der Wirkstoff einem Träger zugesetzt wird, der, in Abwesenheit von Wasser für eine äußerliche Anwendung geeignet ist, für die Behandlung bakterieller Infektionen der Haut und der Schleimhäute (Bindehaut, Mund- und Rachenschleimhaut, Vulvo-Vaginal-Zervical-Schleimhaut).

2. Verfahren nach Anspruch 1 für die Herstellung einer Salbe, die bezogen auf 100 g, folgende Rezeptur aufweist:

| | | | | | |
|---|---|---|---|---|---|
| Miocamycin | von | 1 | bis | 5 | g |
| Äthylzellulose | von | 0,08 | bis | 0,4 | g |
| Nicht-ionische Tenside | von | 3 | bis | 7 | g |
| Aliphatische Mischester | von | 50 | bis | 80 | g |
| Glyzeride gesättigter Fettsäuren | von | 5 | bis | 15 | g |
| Konservierungsmittel | von | 0,05 | bis | 0,2 | g |

3. Verfahren nach Anspruch 1 für die Herstellung einer pharmazeutischen Zusammensetzung in Form von Kügelchen (Globuli), welche die folgende Einheitenrezeptur aufweist:

| | | | | | | |
|---|---|---|---|---|---|---|
| Miocamycin | von | 0,25 | bis | 0,8 | g |
| Ähtylzellulose | von | 0,02 | bis | 0,07 | g |
| Nicht-ionische Tenside | von | 0,05 | bis | 0,15 | g |
| Polyäthylenglycole | von | 3 | bis | 6 | g |
| Propylenglycol | von | 0 | bis | 2 | g |

4. Verfahren nach Anspruch 1 für die Herstellung einer pharmazeutischen Zusammensetzung in Form von Kügelchen (Globuli), welche die folgende Einheitenrezeptur aufweist:

| | | | | | | |
|---|---|---|---|---|---|---|
| Miocamycin | von | 0,25 | bis | 0,8 | g |
| Ähtylzellulose | von | 0,02 | bis | 0,07 | g |
| Halbsynthetische Glyzeride von gesättigten Fettsäuren | von | 3 | bis | 6 | g |

5. Verfahren nach Anspruch 1 für die Herstellung einer pharmazeutischen Zusammensetzung in Form einer Augensalbe, die, bezogen auf 100 g, folgende Rezeptur aufweist:

| | | | | | | |
|---|---|---|---|---|---|---|
| Miocamycin | von | 1 | bis | 10 | g |
| Äthylzellulose | von | 0,08 | bis | 0,8 | g |
| Pflanzliche Öle | von | 20 | bis | 30 | g |
| Festes Parafin | von | 40 | bis | 60 | g |
| Nicht-ionische Tenside | von | 0,5 | bis | 5 | g |
| Polyoxyäthylenierte Ölsäureglyzeride | von | 5 | bis | 10 | g |
| Konservierungsmittel | von | 0,05 | bis | 0,2 | g |

6. Verfahren für die Herstellung einer pharmazeutischen Zusammensetzung, die Miocamycin als Wirkstoff enthält, wobei der Wirkstoff einem für eine äußerliche Anwendung geeigneten Träger zugefügt wird, welche Zusammensetzung die Form einer Mund-spülung aufweist-und-die aus-einem,Granulat in einem Einzeldosispaket besteht, das zum Zeitpunkt des Gebrauches in Wasser zu suspendieren ist und die die folgende Einheitsrezeptur aufweist:

| | | | | | | |
|---|---|---|---|---|---|---|
| Miocamycin | von | 0,3 | bis | 0,9 | g |
| Äthylzellulose | von | 0,024 | bis | 0,08 | g |
| Hydroxypropylmethyl-zellulose | von | 0,15 | bis | 0,3 | g |
| Mannit | von | 1,5 | bis | 4 | g |
| Geschmacksstoffe | von | 0,05 | bis | 0,15 | g |
| Dimethicone | von | 0,0005 | bis | 0,001 | g |
| Nicht-ionische Tenside | von | 0,0005 | bis | 0,001 | g |
| Synthetischer Süßstoff | von | 0,005 | bis | 0,05 | g |

7. Verfahren zur Herstellung einer pharmazeutischen Zusammensetzung, die Miocamycin als Wirkstoff enthält und für äußerliche Anwendung bei der Behandlung bakterieller Infektionen der Haut und der Schleimhäute bestimmt ist, wobei der Wirkstoff in die Kappe einer Flasche eingefüllt wird, die Flasche Wasser und ein Geliermittel enthält und der Wirkstoff mit dem Inhalt der Flasche zum Zeitpunkt des Gebrauches gemischt wird.

8. Verfahren nach An spruch 7 für die Herstellung einer Zusammensetzung, welche folgende, auf 100 g bezogene Rezeptur hat:

Inhalt der Flasche:

```
Hydroxypropylmethylzellulose    von   0,5   bis     3   g
Poloxamere und/oder Poloxamine von   5     bis    50   g
Konservierungsmittel            von   0,05  bis    0,2  g
Puffersubstanzen ad pH 7 - 7,5                 .
Wasser                                     ad  100   g
Inhalt der Behälterkappe:
Miocamycin                      von   1     bis     5   g
Äthylzellulose                  von   0,08  bis    0,4  g
```

**Revendications**

**Revendications pour les Etats contractants suivants : AT, BE, CH, DE, FR, GB, LI, LU, NL, SE**

1. Composition pharmaceutique stable contenant de la miocamycine en tant que principe actif et un excipient convenant à l'administration topique, en l'absence d'eau, pour le traitement d'infections bactériennes touchant la peau et les membranes muqueuses (conjonctivales, buccopharyngiennes et vulvo-cervico-vaginales).

2. Composition selon la revendication 1, sous forme de pommade ayant une formulation comme suit, rapportée à 100 g :

```
Miocamycine                          de   1    à    5   g
Ethylcellulose                       de   0,08 à   0,4 g
Agents tensioactifs non ioniques     de   3    à    7   g
Esters mixtes aliphatiques           de  50    à   80   g
Glycérides des acides gras saturés   de   5    à   15   g
Conservateurs                        de   0,05 à   0,2 g
```

3. Composition selon la revendication 1, sous la forme de globules ayant la formulation unitaire suivante :

```
Miocamycine                          de 0,25 à 0,8   g
Ethylcellulose                       de 0,02 à 0,07 g
Agents tensioactifs non ioniques     de 0,05 à 0,15 g
Polyéthylène-glycol                  de 3    à 6     g
Propylène-glycol                     de 0    à 2     g
```

4. Composition selon la revendication 1 sous la forme de globules ayant la formulation unitaire suivante :

| | |
|---|---|
| Miocamycine | de 0,25 à 0,8 g |
| Ethylcellulose | de 0,02 à 0,07 g |
| Glycérides semi-synthétiques des acides gras saturés | de 3 à 6 g |

5. Composition selon la revendication 1, sous la forme d'une pommade ophtalmique ayant la formulation suivante pour 100 g :

| | |
|---|---|
| Miocamycine | de 1 à 10 g |
| Ethylcellulose | de 0,08 à 0,8 g |
| Huiles végétales | de 20 à 30 g |
| Paraffine solide | de 40 à 60 g |
| Agents tensioactifs non ioniques | de 0,5 à 5 g |
| Glycérides oléiques polyoxy-éthylénées | de 5 à 10 g |
| Conservateurs | de 0,05 à 0,2 g |

6. Composition pharmaceutique contenant de la miocamycine en tant que principe actif pour l'administration topique sous forme de collutoire consistant en granules dans un paquet de dose unique, à mettre en suspension dans l'eau au moment de l'utilisation, ayant la formulation unitaire suivante :

| | |
|---|---|
| Miocamycine | de 0,3 à 0,9 g |
| Ethylcellulose | de 0,024 à 0,08 g |
| Hydroxypropylméthylcellulose | de 0,15 à 0,3 g |
| Mannitol | de 1,5 à 4 g |
| Agents de saveur | de 0,05 à 0,15 g |
| Diméthicone | de 0,0005 à 0,001 g |
| Agents tensioactifs non ioniques | de 0,0005 à 0,001 g |
| Edulcorants synthétiques | de 0,005 à 0,05 g |

7. Composition pharmaceutique contenant de la miocamycine pour l'administration topique pour le traitement d'infections bactériennes de la peau et des membranes muqueuses consistant en un flacon contenant de l'eau et un agent gélifiant et un capuchon contenant la miocamycine en tant que principe actif, le principe actif étant mélangé au contenu du flacon au moment de l'utilisation.

8. Composition selon la revendication 7, ayant une formulation comme suit, rapportée à 100 g : Contenu du flacon :

| | |
|---|---|
| Hydroxypropylméthylcellulose | de 0,5 à 3 g |
| Poloxamères et/ou poloxamines | de 5 à 50 g |
| Conservateurs | de 0,05 à 0,2 g |
| Substances tampon q.s.p | pH 7 - 7,5 |
| Eau | q.s.p. 100 g |

Contenu du capuchon réservoir :

| | | | |
|---|---|---|---|
| Miocamycine | de 1 | à | 5 g |
| Ethylcellulose | de 0,08 | à | 0,4 g |

**Revendications pour les Etats contractants suivants : GR, ES**

1. Procédé pour la préparation d'une composition pharmaceutique stable contenant de la miocamycine en tant que principe actif, dans lequel on ajoute le principe actif à un excipient convenant à l'administration topique, en l'absence d'eau, pour le traitement d'infections bactériennes touchant la peau et les membranes muqueuses (conjonctivales, bucco-pharyngiennes, et vulvo-cervico vaginales).

2. Procédé selon la revendication 1, pour la préparation d'une pommade ayant la formulation suivante, rapportée à 100 g :

| | | | |
|---|---|---|---|
| Miocamycine | de 1 | à | 5 g |
| Ethylcellulose | de 0,08 | à | 0,4 g |
| Agents tensioactifs non ioniques | de 3 | à | 7 g |
| Esters mixtes aliphatiques | de 50 | à | 80 g |
| Glycérides des acides gras saturés | de 5 | à | 15 g |
| Conservateurs | de 0,05 | à | 0,2 g |

3. Procédé selon la revendication 1 pour la préparation d'une composition pharmaceutique sous forme de globules ayant la formulation unitaire suivante :

| | | | |
|---|---|---|---|
| Miocamycine | de 0,25 | à | 0,8 g |
| Ethylcellulose | de 0,02 | à | 0,07 g |
| Agents tensioactifs non ioniques | de 0,05 | à | 0,15 g |
| Polyéthylène-glycol | de 3 | à | 6 g |
| Propylène-glycol | de 0 | à | 2 g |

4. Procédé selon la revendication 1, pour la préparation d'une composition pharmaceutique sous forme de globules ayant la formulation unitaire suivante :

| | | | |
|---|---|---|---|
| Miocamycine | de 0,25 | à | 0,8 g |
| Ethylcellulose | de 0,02 | à | 0,07 g |
| Glycérides semi-synthétiques des acides gras saturés | de 3 | à | 6 g |

5. Procédé selon la revendication 1, pour la préparation d'une composition pharmaceutique sous la forme d'une pommade ophtalmique ayant la formulation suivante pour 100 g :

| Miocamycine | de | 1 | à | 10 | g |
|---|---|---|---|---|---|
| Ethylcellulose | de | 0,08 | à | 0,8 | g |
| Huiles végétales | de | 20 | à | 30 | g |
| Paraffine solide | de | 40 | à | 60 | g |
| Agents tensioactifs non ioniques | de | 0,5 | à | 5 | g |
| Glycérides oléiques polyoxy-éthylénées | de | 5 | à | 10 | g |
| Conservateurs | de | 0,05 | à | 0,2 | g |

6. Procédé pour la préparation d'une composition pharmaceutique contenant la miocamycine en tant que principe actif dans lequel le principe actif est ajouté à un excipient convenant à l'administration topique, sous la forme d'un collutoire constitué par des granules dans un paquet à dose unique, à mettre en suspension dans l'eau au moment d'utilisation et ayant la formule unitaire suivante :

| Miocamycine | de | 0,3 | à | 0,9 | g |
|---|---|---|---|---|---|
| Ethylcellulose | de | 0,024 | à | 0,08 | g |
| Hydroxypropylméthylcellulose | de | 0,15 | à | 0,3 | g |
| Mannitol | de | 1,5 | à | 4 | g |
| Agents de saveur | de | 0,05 | à | 0,15 | g |
| Diméthicone | de | 0,0005 | à | 0,001 | g |
| Agents tensioactifs non ioniques | de | 0,0005 | à | 0,001 | g |
| Edulcorants synthétiques | de | 0,005 | à | 0,05 | g |

7. Procédé pour la préparation d'une composition pharmaceutique contenant de la miocamycine en tant que principe actif, convenant pour l'administration topique pour le traitement d'infections bactériennes touchant la peau et les membranes muqueuses, dans lequel le principe actif est introduit dans le capuchon d'un flacon, le flacon contenant de l'eau et un agent gélifiant, le principe actif étant mélangé au contenu du flacon au moment de l'utilisation.

8. Procédé selon la revendication 7, pour la préparation d'une composition ayant une formulation comme suit, rapportée à 100 g :

Contenu de la bouteille :

| Hydroxypropylméthylcellulose | de | 0,5 | à | 3 | g |
|---|---|---|---|---|---|
| Poloxamères et/ou polaxamines | de | 5 | à | 50 | g |
| Conservateurs | de | 0,05 | à | 0,2 | g |
| Substances tampon q.s.p. | pH | 7 - 7,5 | | | |
| Eau | | | q.s.p. | 100 | g |

Contenu du capuchon réservoir :

| Miocamycine | de | 1 | à | 5 | g |
|---|---|---|---|---|---|
| Ethylcellulose | de | 0,08 | à | 0,4 | g |